# EUROPEAN PATENT APPLICATION

(11) **EP 0 778 352 A1**
(43) Date of publication of application: **11.06.1997**
(21) Application number: 97100006.2
(22) Date of filing: 15.12.1989
(51) Int. Cl.: C12Q 1/68, C12Q 1/70, C07H 21/04

(54) **Self-sustained, sequence replication system**

(30) Priority: 16.12.1988 US 285467
(62) Divisional of application: 89313154.0
(71) Applicant: SISKA DIAGNOSTICS, INC., La Jolla, CA 92037 (US)
(72) Inventor: Gingeras, Thomas Raymond, Encinitas, California 92024 (US); Guatelli, John C., San Diego, California 92117 (US); Whitfield, Kristina Marie, Carlsbad, California 92009 (US)
(74) Representative: Baldock, Sharon Claire

(57) **Abstract**

The present disclosure provides details of an invention comprising an amplification system for the detection of target nucleic acids, most particularly target nucleic acid sequences, in an isothermal setting wherein all of the reagents necessary to conduct the amplification are present and the reactions are self-sustaining and continuous. Featured is a selective enzymatic digestion of a RNA/DNA duplex, formed by hybridization of a DNA primer with target nucleic acid, thus freeing the DNA strand for further hybridization followed by primer extension to provide a DNA duplex that can serve as a template for production of a plurality of transcripts that can be recycled and/or detected as such for deduced presence of target nucleic acid sequence.

## Description

### Related Applications

Reference is made to U.S. Patent Application Serial No. 07/064141, filed 19 June 1987, and a continuing application thereof, U.S. Serial No. 07/202978, filed 6 June 1988 and published as PCT International Publication No. WO 88/10315, the entire disclosures of each of which applications is hereby incorporated by express reference.

### Field of the Invention

The present invention relates generally to advances in molecular biology and recombinant DNA technology.

More particularly, the present invention is directed to methods and means, including assays and pharmaceutical kits containing requisite reagents and means, for detecting in an in vitro or ex vivo setting the presence in a biological sample of a target nucleic acid sequence, or of an extrapolated RNA sequence from a corresponding target DNA sequence, and by deduction of corresponding polypeptide that RNA (DNA) sequence encodes.

The present invention features the provision of providing the amplification of such a particular nucleic acid target sequence in a self-sustained, single-pot in vitro system wherein the amplification of the target nucleic acid sequence is accomplished by means of the preparation of multiple transcript products that have the optional capability of self-replication. This self-sustained amplification system avoids the necessity of repeated denaturation of nucleic acid duplexes that require temperature cycling. The present invention combines in a novel manner, all of the reagents necessary to form in a single reaction setting an amplified product, or a product suitable for detection in amplified form, representing the presence of a target nucleic acid sequence.

Among the applications in which the present invention finds use are in analyses of RNA sequences, or by extrapolation, DNA sequences, that are characteristic of a particular or general pathogenic disease or condition by the in vitro or ex vivo nucleic acid probe hybridization assays of body fluids and tissues containing requisite target nucleic acid sequence(s).

### Background of the Invention

It is a goal in this art to detect various nucleic acid sequences in a biological sample, in which a given sequence, a so-called target nucleic acid, is present in small amounts relative to its existence amongst a wide variety of other nucleic acid species including RNA, DNA or both. Thus, it is desirable to detect the nucleic acid encoding polypetides that may be associated with pathological diseases or conditions, such as, for example, nucleic acid correlating to that of the human immunodeficiency virus (HIV-1). In addition to the detection of nucleic acids encoding such polypeptides, it is desirable to detect other nucleic acids characteristic of a pathological disease or condition such as a defective gene, as in the case of the detection of a defective human betaglobin gene as exemplified in hemophilia.

Characteristically, the nucleic acids associated with such are present, if at all, in very small amounts relative to total nucleic acid in a given biological sample, such as blood or other body fluid or tissue sample of a given individual to be tested. The detection of such nucleic acid species requires such specificity that, if present, it is detectable and measurable from amongst the wide variety of other nucleic acid species with which it is environmentally associated. Some of these species may bear close homology, at least in isolated segments, with the target nucleic acid. Further, as noted above, these target nucleic acid species are very often found only in very minute amounts in the biological sample being tested. And yet, for proper diagnosis of the underlying disease state, it is essential that even small amounts of such target nucleic acid be detectable unequivocally for fidelity of the assay system.

Several approaches have been advanced for accomplishing the goal of the art. In one, the amount of nucleic acid in the sample is not altered or affected. Instead, a reporter system is developed whereby a large number of detectable molecules corresponding to the nucleic acid target are produced for ready detectability and measurement. Such a reporter system is a signal-generating system associated with the target nucleic acid producing a detectable signal representative of the number of molecules of target sequence.

Another approach has been developed that is fundamentally different in that it involves increasing the copy number of the target nucleic acid sequence itself. This can be done by selective amplification of the target nucleic acid sequence. One can refine the culture techniques of the sample such that somehow the target nucleic acid sequence is amplified preferentially to other nucleic acid sequences. These techniques are cumbersome and time consuming and subject to trial and error.

Another example of this approach is amplification of a target nucleic acid sequence in a so-called "polymerase chain reaction" (PCR). This technique was reported by Saiki et al., Science 230, 1350 (1985) and Mullis et al., European Patent Application Publication Nos. 200362 and 201184 (See also U.S. Patents 4683195 and 4683202), and particularly entails (1) hybridizing to a segment of target nucleic acid sequence a primer, (2) extending said primer with a polymerase, and (3) rendering single-stranded the duplexes resulting from the primer extension reaction. This procedure can be repeated over a number of cycles so as to amplify the underlying target nucleic acid sequence.

An improved, novel approach is detailed in U. S. Application Serial Nos. 07/064141 and 07/202978 and PCT International Publication No. WO 88/10315, supra. It employs a novel RNA transcript production step in conjunction with, and derival from, a synthesized double-stranded cDNA copy of the target sequence operably linked to a promoter therefor. By virtue of the transcription step being the dominant aspect of novelty, it is conveniently referred to as a transcription-based amplification system (TAS).

Thus, that invention involves the in vitro or ex-vivo detection of at least one specific nucleic acid sequence (target sequence or segment) in a sample containing nucleic acid, comprising a method of preparing a double-stranded nucleic acid containing a sequence corresponding to a target sequence operably linked to a RNA-polymerase promoter therefor, employing said double-stranded nucleic acid as a double-stranded nucleic acid template for the preparation of a plurality of RNA transcripts therefrom, each bearing an RNA sequence corresponding to said target sequence, and detecting the presence of said RNA sequence and by analogy the presence of target sequence.

The double-stranded nucleic acid template, in turn, is prepared by providing a first nucleic acid primer or probe containing a promoter sequence operably linked to a sequence corresponding to a segment of a target sequence, hybridizing under suitable conditions said first nucleic acid primer with target sequence in a sample containing nucleic acid, extending the hybridized said first nucleic acid primer in a polymerase extension reaction complementarily to the target sequence to form a corresponding duplex nucleic acid, separating the strands of said duplex, hybridizing to the separated promoter containing sequence strand under suitable conditions a second nucleic acid primer at the end opposite said promoter sequence, and extending the hybridized said second nucleic acid primer in a polymerase extension reaction complementarily to said promoter containing sequence.

Thus, that invention yields a single-stranded RNA transcript, or an RNA-DNA duplex formed therefrom when measures are not undertaken to prevent its formation that has a sequence corresponding to the target nucleic acid. The single-stranded RNA transcript product is struck-off more or less continuously and provides for direct detection of target segment without the necessity of cumbersome, error-prone repeated PCR cycles and strand separation. Such advantages are not provided by the PCR technique that yields double-stranded DNA (one strand of which comprises target segment and the other strand of which comprises complement of target segment) that need to be separated before detection and only after a large number of repeated cycles necessary to reach acceptable amplification levels.

It is in object of the present invention as a selective embodiment to take further advantage of the basic replicative process for amplification, for ease in the detection of target nucleic acid sequences, thus achieving exponential copying without the requirement of temperature cycling and otherwise monitoring the course of the amplification method in respect of reagent additions, etc. It is a further object of the present invention to combine in a novel manner the advantages of the transcription and extension product procedures as a means for detecting and measuring corresponding target nucleic acid.

It is a basic object of the present invention to employ a selective digestion enzymatically of the RNA strand of a RNA/DNA duplex, formed by hybridizing a primer to a nucleic acid target sequence followed by primer extension, as a means of providing the DNA strand as a template for further hybridization thereto followed by primer extension. The product double-stranded DNA duplex contains at least one promoter sequence that is recognizable by a DNA-dependent RNA polymerase and thus serves as a template for the production of a plurality of transcripts that are ultimately detected and measured as a means of detecting and measuring target nucleic acid sequence. This object provides the advantages of target sequence amplification that is self-sustained without the necessity of temperature cycling in a single reaction mixture containing (three) appropriate enzymatic activities and at least one primer containing a promoter operatively recognizable by a DNA-dependent RNA polymerase.

It is thus an overall object of the present invention to meet the goals enumerated by the art and to provide selective means to further advantage amplification of target nucleic acid sequences. It further provides a straightforward technique that can be utilized reproducibly in an acceptably short period of time, in an isothermal reaction system, employing the convenience of known reagents and having the precision necessary to reach consistent scientific results; one that can be employed in a reproducible assay setting and that is adaptable for use in kits for laboratory/clinical analyses.

### Summary of the Invention

The present invention is predicated on the use of a means to "strand-separate" a RNA/DNA duplex so as to free the DNA strand thereof for hybridization with obligonucleotides containing RNA polymerase binding sequences (PBS) followed by primer extension so as to form a DNA duplex that can serve as a template for the preparation of plurality of corresponding RNA transcripts which are susceptible to detection and measurement as a deduced assay for the presence of target nucleic acid sequence. The RNA/DNA duplex is in turn produced by hybridization to a RNA target of a DNA primer operatively containing a promoter sequence, followed by primer extension.

The means of the present invention for causing "strand-separation" involves the use of an enzyme having RNase H-like activity, such as RNase H, which will selectively and preferentially digest the RNA strand of the duplex so as to free the DNA strand for further processing. The use of such an enzyme eliminates the use of a temperature cycle to denature said duplex.

The nucleic acid target sequence can be one present intrinsically as such in a nucleic acid sample, or it can be a corresponding DNA target sequence extrapolation product. The extrapolation product is prepared by denaturing double-stranded DNA target sequence, hybridizing to it a primer sequence having an operatively associated promoter sequence followed by a primer extension reaction to form a DNA duplex. This DNA/DNA duplex is in turn denatured and the strand containing the promoter sequence hybridized at its end opposite the promoter with a second primer followed by primer extension so as to form a DNA duplex which, when contacted with a DNA-dependent RNA polymerase produces the corresponding transcript, extrapolation product. The RNA then serves as target nucleic acid sequence for purposes of the present invention.

After the nucleic acid target sequence, whatever its source, has been provided, in accordance with the present invention it is hybridized with a primer sequence operatively associated with a promoter sequence, followed by primer extension to produce a RNA/DNA duplex containing a promoter sequence at the 5'-end of the DNA strand. The primer extension reaction can be conducted with any suitable polymerase, such as reverse transcriptase. The basic aspect of the present invention then serves to free the DNA strand of the RNA/DNA duplex by treatment of the RNA/DNA duplex with an enzyme that selectively digests the RNA strand, such as RNase H. The thus freed DNA strand either 1) undergoes self-generated primer extension via a RNA primer resulting from the previous selective digestion or 2) is hybridized with an extrinsically derived oligonucleotide primer optionally bearing operatively a promoter sequence. Primer extension creates a double-stranded DNA duplex containing one or two promoter sequences, that serves as a template for DNA-dependent RNA polymerase induction of transcription to give a plurality of transcripts.

Given that the foregoing reaction sequence can be performed isothermally and in contemporaneous mixture with the three appropriate enzyme activities, such as is provided reverse transcriptase, RNase H and DNA-dependent RNA polymerase, for example, the various steps in the foregoing process are done in a continuous, simultaneous fashion over a given period of time. Thus, at the point provided above as an end point, the produced transcripts can be detected and measured for deduced presence of starting target nucleic acid sequence. However, given the continuous and simultaneous nature of the above-described reaction sequence being independent of temperature cycling and requiring only a single, initial addition of enzyme activities required to carry out these reactions, the transcript products themselves undergo hybridization with a primer optionally bearing operatively an additional promoter sequence. This hybridization complex is followed by primer extension reaction to produce a second RNA/DNA duplex that, in turn, is subjected to the action of the selective RNA digestion enzyme to free the DNA strand therefrom. It is in turn hybridized with a self-generated RNA primer or extrinsically derived oligonucleotide primer present in the reaction mixture that may optionally bear operatively a promoter sequence so as to produce a second DNA duplex that is susceptible to recognition by a DNA-dependent RNA polymerase to produce a plurality of transcripts having a sense opposite the transcripts produced initially.

While the mechanism of the foregoing reaction sequence(s) has not been fully elucidated, it is believed that because the reaction mixture employs in combination the three appropriate enzyme activities (such as is provided by reverse transcriptase, RNase H and DNA-dependent RNA polymerase, for example) and at least one primer containing a promoter recognized by the polymerase, and because the reactions are not dependent on a temperature cycle, it is contemplated that where two promoter-containing oligonucleotide primers are used, the reactions may go through several cycles spontaneously and continuously, producing both sense and anti-sense transcripts that may variously be detected and measured to provide an amplification assay of the amount of target nucleic acid sequence present in the sample-tested.

The essence of the present invention provides for a reaction mixture that is permitted essentially to remain dormant for a period of time at a suitable temperature, with no need for cycling between higher and lower temperatures and no need for periodic addition of enzyme or other reagents, whereby a target nucleic acids sequence is amplified continuously and spontaneously in a self-generated fashion in the presence of at least one primer bearing operatively a promoter sequence and enzyme activity such as is provided by RNA polymerase that recognizes the polymerase binding site of the promoter, a reverse transcriptase, an enzyme such as RNase H that selectively digests RNA when that RNA is hybridized to DNA in duplex form, and requisite nucleoside triphosphate substrates for the RNA polymerase and reverse transcriptase. In such a system as defined herein, reproducible amplification levels as high as 10⁷ can be achieved in approximately two hours at about 37° C, using for example T7 RNA polymerase, AMV reverse transcriptase and E. coli RNase H. A temperature between about 4° C and about 50° C, preferable in the range around 40° C, is operable. The size of the nucleic acid target sequence in preferred embodiments, contains fewer than about 250 bases. Other variables may affect the optimization of the amplification herein such as the RNA polymerase employed, the reverse transcriptase employed, pHs, salt concentrations, nucleoside triphosphate concentrations. These variables are within the ordinary ken of the skilled artisan.

Thus, the present invention involves the in vitro or ex-vivo detection of at least one specific nucleic acid target sequence in a sample containing a heterogeneous collection of RNAs. The present invention reduces to a method comprising preparing a double-stranded DNA encoding a sequence corresponding to a target sequence and having an operative RNA polymerase promoter, said double-stranded DNA being prepared, in turn, by hybridization followed by primer extension to a DNA strand that has been freed from its RNA complement in a RNA/DNA duplex by action of a RNA selective digestion enzyme, said RNA/DNA duplex having been formed by hybridization with a primer bearing operatively a promoter sequence to a target nucleic acid sequence followed by primer extension. The double-stranded DNA serves as a template for the preparation of a plurality of RNA transcripts therefrom, each bearing an RNA sequence corresponding to said target nucleic acid sequence. The presence of said RNA sequence and by deduction the presence of target sequence, can be detected and measured.

The present invention is directed to all associated methods and means to prepare and use such RNA transcripts. In an embodiment, the present invention is directed to the optionally repetitive method of preparing said double-stranded nucleic acid template defined above providing a first nucleic acid primer containing a promoter sequence operatively linked to a sequence corresponding to a segment of a target nucleic acid sequence, hybridizing under suitable conditions said first nucleic acid primer with target nucleic acid sequence in a sample containing nucleic acid, extending the hybridized said first nucleic acid primer in a polymerase extension reaction complementarily to the target sequence to form a corresponding RNA/DNA duplex nucleic acid, enzymatically cleaving the RNA of said RNA/DNA duplex, hybridizing to the freed promoter-containing DNA sequence strand under suitable conditions a second nucleic acid primer at the end opposite said promoter sequence either via 1) a product derived RNA primer or 2) an oligonucleotide primer optionally containing a promoter sequence operatively linked thereto, extending the hybridized said second nucleic acid primer in a polymerase extension reaction complementarily to said promoter-containing sequence.

The present invention in a further embodiment is directed to methods and means of employing said double-stranded nucleic acid supra., as a template for the preparation of a plurality of RNA transcripts therefrom in a reaction catalyzed by a DNA-dependent RNA polymerase that recognizes the promoter thereof, and after further optional cycling as described above, detecting and measuring the presence of said RNA transcripts.

The present invention in a further embodiment, is directed to the improvement in the method of amplifying a target nucleic acid sequence, generated as such or as an extrapolation product from a target DNA sequence, comprising the steps of hybridizing with said target sequence a DNA primer having a promoter sequence operatively linked thereto followed by primer extension to give a corresponding RNA/DNA duplex, hybridization of the freed DNA extension product strand bearing the promoter sequence of said duplex with a second primer at the end opposite the promoter sequence followed by primer extension to form a double-stranded DNA template useful for preparation of optionally replicatable RNA transcripts therefrom for detection as such or for recycling as defined above. The improvement comprises freeing the extension product DNA strand bearing the promoter sequence of said RNA/DNA duplex primer by selective enzymatic digestion of the RNA strand of said RNA/DNA duplex.

In an embodiment, the present invention is directed to the product of the process of treating a RNA/DNA duplex RNA, having linked at the 5'-end of the DNA sequence a promoter sequence, with a selective RNA digestion enzyme.

In a further embodiment, the present invention is directed to kits comprising requisite reagents and associated means useful in the in vitro or ex vivo detection of at least one specific nucleic acid target sequence in a sample containing RNA, employing the methods and means defined supra.

### Detailed Description of the Invention

### 1. Brief Description of the Drawings

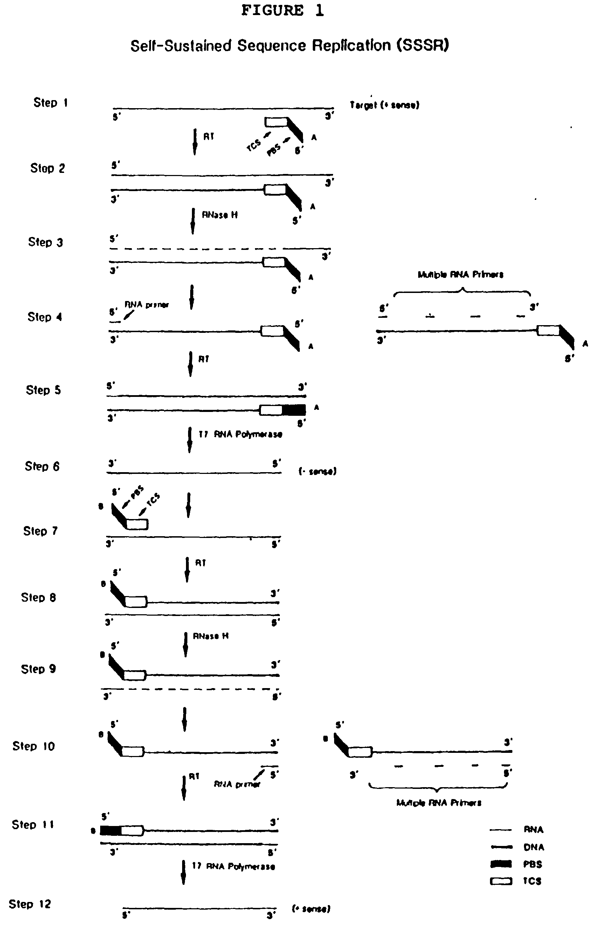

Figure 1 represents a schematic representation of an embodiment of the present invention. The schematic representation provides for a total of 12 steps which, in preferred aspects hereof, are thought to be continuous, self-generated steps upon presence of the requisite three enzymes in the reaction mixture together with target sequence and a given operable temperature. The three enzymes are as listed, e.g., reverse transcriptase (RT) used for primer extension reaction, RNase H which is a selective RNA digestion enzyme, representing the basic aspect of the present invention, and T7 RNA polymerase as an example of a useful enzyme for preparing transcripts from the DNA duplex template. The essence of the invention, as noted above, is represented by step three of the schematic representation and it is understood and contemplated that the RNA transcript product of step six may be detected and measured as such or it may be subjected to continuous reaction as listed in steps 7 through 12 to form the antisense strand of an RNA transcript. The generated RNA transcripts are detected and measured as a consequential result of the presence of the target nucleic acid sequence. PBS represents the polymerase binding site of the promoter sequence. TCS represents target complementary sequence.

### 2. General Methods and Definitions

Reference is made to standard textbooks of molecular biology that contain definitions and methods and means for carrying out basic techniques of the present invention, such as:
DNA probe or primer preparation, including DNA synthesis or isolation of sequences from natural source via restriction enzyme cleavage and the tailoring thereof so as to be suitable as such or when linked to other DNA for use as a primer or probe herein;
preparation of oligonucleotides with different functional sequences for use in hybridization;
hybridization methodology including variations in stringency conditions for producing more or less hybridization certainty depending on the degree of homology of the primer to a target DNA sequence;
identification, isolation or preparation of promoters, or more specifically promoters or sites recognized by bacteriophage DNA-dependant RNA polymerase and bacteriophage RNA-dependant RNA polymerase or in the employment of eukaryotic systems, viral DNA- and RNA-dependent RNA polymerase, for example, adenovirus-encoded RNA polymerase and brome mosaic virus RNA polymerase;
identification, isolation or preparation of RNA polymerase capable of recognizing said promoters referred to above or capable of primer extension reactions; conditions conducive to the production of RNA transcripts, including so-called transcription-enhancer sequences;
conditions conducive to the initiation and maintenance of primer extension reactions including use of DNA dependent polymerase and dNTPs;
the mechanism and methodology for (induced) replication; and so forth.

See, for example, Maniatis et al., Molecular Cloning: A Laboratory Manual, Cold Spring Harbor Laboratory, New York 1982), and Colowick et al., Methods in Enzymology Vol. 152, Academic Press, Inc. (1987), and the various references cited therein; Hong, Bioscience Reports 1, 243 (1981); Cooke et al., J. Biol. Chem. 255 6502 (1980); and Zoller et al., Methods in Enzymology 100, 468-500 (1983); Crea et al., nucleic acids Res. 8, 2331 (1980); Narang et al., Meth. Enzyme. 68, 90 (1979); Beaucage et al., Tetrahedron Letters 22, 1859 (1981); Brown et al., Meth. Enzym. 68,109 (1979); Caruthers et al., Meth. Enzym. 154, 287 (1985); Hitzeman et al., J. Biol. Chem. 255, 2073 (1980); Lee et al., Science 239, 1288 (1988); Milligan et al., nucleic acids Res. 15, 8783 (1987); Miller et al., Virology 125, 236 (1983), Ahlquist et al., J. Mol. Biol. 153, 23 (1981); Miller et al., Nature 313, 68 (1985); Ahlquist et al., J. Mol. Biol. 172, 369 (1984); Ahlquist et al., Plant Mol. Biol. 3, 37 (1984); Ou et al., PNAS 79, 5235 (1982); Chu et al., Nucl. Acids Res. 14, 5591 (1986); European Patent Application Publn. No. (EPA) 194809; Marsh et al., Positive Strand RNA Viruses, p. 327-336, Alan R. Liss (publ.; New York) 1987; proceedings of UCLA Symposium, 1986); Miller et al., J. Mol. Biol. 187, 537 (1986); Stoflet et al., Science 239, 491 (1988); Kramer et al., J. Mol. Biol. 89, 719 (1974); Saris et al., Nucl. Acids Res. 10, 4831 (1982); Bresser et al., PNAS 80, 6523 (1983); Chu et al., nucleic acids Research 16, 3671 (1988), Gubler et al., Gene 25, 263 (1983) and D'Alessio et al., nucleic acids Res. 16, 1999 (1988), as well as the references cited therein.

All of the aforecited publications are by this reference hereby expressly incorporated by reference herein.

By the term "promoter" is meant a nucleic acid sequence (naturally occurring or synthetically produced or a product of restriction digest) that is specifically recognized by an RNA polymerase that binds to a recognized sequence and initiates the process of transcription whereby an RNA transcript is produced. It may optionally contain nucleotide bases extending beyond the actual recognition site, thought to impart additional stability toward degradation processes, and may also include additional plus (+) nucleotides contiguous to the transcription initiation site. In principle, any promoter sequence may be employed for which there is a known and available polymerase that is capable of recognizing the initiation sequence. Typical, known and useful promoters are those that are recognized by certain bacteriophage polymerase such as bacteriophage T3, T7 or SP6. See Siebenlist et al., Cell 20, 269 (1980). These are but examples of those polymerases that can be employed in the practice of the present invention in conjunction with their associated promoter sequences.

The "RNA transcript" hereof is the ribonucleic acid sequence produced after transcription initiation following RNA polymerase recognition of the promoter sequence (see supra). The production of such transcripts is more or less continuous, dependent in part on the amount of polymerase present.

By the term "probe" or "primer" in the present context is meant a single-stranded nucleic acid sequence (naturally occurring or synthetically produced or a product of restriction digest) that has sufficient complementarity with the target sequence such that under suitable hybridization conditions it is capable of hybridizing, that is binding to, the appropriate (target) sequence. A typical probe or primer is at least about 10 nucleotides in length, and most preferably is of approximately 20 or more nucleotide bases in length, and in its most preferred embodiments, it shares identity or very high complementarity with the appropriate (target) sequence. See, for example, EPA 128042 (publd. 12 Dec 84). Such probe or primer is such that it will hybridize to a complement sequence ford purposes of a primer extension reaction in the presence of appropriate reagents and conditions.

The term "operatively linked", or "associated" or grammatical variations thereof, in particular in connection with the linkage of a promoter sequence within an RNA encoding DNA sequence, refers to its functionality in producing corresponding RNA transcripts when the promoter is recognized by the suitable polymerase--see supra.

The techniques of forming a detection signal such as via radioactive labeling or chromogenic means using a chromogenic susceptible enzyme are also well known and documented in the art.

A sample on which the assay method of the invention is carried out can be a raw specimen of biological material, such as serum or other body fluid, tissue culture medium or food material. More typically, the method is carried out on a sample which is a processed specimen, derived from a raw specimen by various treatments to remove materials that would interfere with detection of target, such as by causing non-specific binding of affinity molecules. Methods of processing raw samples to obtain a sample more suitable for the assay methods of the invention are well known in the art.

The transcripts (RNA) can be detected in a number of different ways:

Detection can be by ultraviolet absorbance of RNA, as, for example, by the method of contact photoprinting (Kutateladze et al., Anal. Biochem. 100, 129 (1979)).

By employing a radioactively labeled ribonucleoside-5'-triphosphate in the reaction (e.g., ³H-labeled or alpha-³²PO₄-labeled), so that the RNA is radioactive, the RNA can be detected, by any of numerous known procedures, by means of its radioactivity.

Biotin or iminobiotin can be incorporated into RNA, which can then be detected by known techniques with an enzyme-avidin or enzyme-streptavidin adduct, which binds to the RNA-bound biotin and catalyzes production of a conveniently detectable chromogen. Incorporation of biotin or iminobiotin can be accomplished by employing UTP that is biotinylated through a spacer to carbon-5 of the uracil moiety as a substrate for the replicase in the replication reaction. Such UTP's are known compounds. Further, it is known that such UTP's are substrates for QB replicase, and that RNAs which include uracils biotinylated through spacer groups joined to the carbon-5 position, due to use of such UTP's in their synthesis, are templates for QB replicase catalyzed replication.

RNA can be made fluorescent by employing a T4 RNA ligase catalyzed reaction to append nucleotides modified to be fluorescent to the 3'-end of replicative RNA. See Cosstick et al., Nucl. Acids Res. 12, 1791 (1984). The fluorescence of the resulting RNA can be employed to detect the RNA by any of several standard techniques.

Among still other methods that can be used to detect RNA are those wherein a reporter substance, that binds specifically with nucleic acid, is added to the system in which the replication has taken place, or to the medium, such as a positively charged support such as ECTEOLA paper, on which replicated RNA has been isolated, and signal from the reporter substance measured. Such substances include: chromogenic dyes, such as "stains all" (Dahlberg et al., J. Mol. Biol. 41, 139 (1969); methylene blue (Dingman et al., Biochemistry 7, 659 (1968), and silver stain (Sammons et al., Electrophoresis 2, 135 (1981); Igloi, Anal. Biochem. 134, 184 (1983); fluorogenic compounds that bind to RNA -- for example, ethidium bromide (Sharp et al., Biochemistry 12, 3055 (1973); Bailey et al., Anal. Biochem. 70, 75 (1976); and fluorogenic compounds that bind specifically to RNAs that are templates for replication by QB replicase -- for example, a phycobiliprotein (Oi et al., J. Cell Biol. 93, 981 (1982); Stryer et al., U.S. Patent No. 4,520,110) conjugated to the viral subunit of QB replicase.

In assays according to the invention, assays are carried out simultaneously under conditions as nearly alike as possible, on both test and control samples. As understood in the art, control samples are similar to test samples but are known to contain either no target or a known quantity of target. A control with no target establishes the "background," below which it is not possible to distinguish samples which contain target from those which do not. By comparing the amount or concentration of RNA produced in an assay of a test sample with the amount or concentration produced with control samples essayed simultaneously, the presence of target in test sample at a level above background can be determined. If control samples with a range of known concentrations of target are employed, the concentration of target in a test sample can be estimated.

The use of a "replicase" for (autocatalytic) induction of replication of the optionally replicatable RNA transcripts of the present invention are generally known in the art. Suitable examples of such replicases that are useful in the present invention include the so-called QB virus replicase that recognizes certain nucleic acid sequence sites at the ends of the given RNA transcript and the so-called brome mosaic virus (BMV) as well as the alpha virus replicases which are thought to recognize nucleic acid sequence sites at the 3' end of a given RNA transcript. These replicases serve to replicate, that is reproduce, the RNA transcripts and complements so as to multiply copies thereof. When such enzyme is present in the reaction during the process of transcription, it can be foreseen that the multiple transcripts that are produced during transcription can themselves undergo replication so as to exponentially increase the amount of RNA transcript product.

### 3. Detailed Description of Embodiments

The quintescense of the present invention is the ability to complete multiple cycles of amplification in vitro without the need for thermal cycling or the addition of supplementary enzymes. The figure appended to the present specification outlines the scheme of a preferred embodiment in diagrammatic fashion. A principal and signal aspect of the present invention is the inclusion of the enzyme RNase H. With further reference to the drawing hereof, steps one and two are identical to those employed in the so-called TAS protocol-cf. the patent applications and PCT International Application to which reference is made at the outset of this application-but at step three, instead of a thermal denaturation step, the RNA/DNA hybrid duplex is "strand-separated" by the selective digestion of the RNA target by use of RNase H. The RNase H activity has a specificity for RNA only when it is present in an RNA/DNA hybrid duplex. The products of this digestion can either be unique RNA oligomers or multiple RNA oligomers (step 4), and in turn, these oligomers can act as primers for DNA synthesis using reverse transcriptase (RT) as the catalyst of this cDNA reaction (step 5). The double-stranded DNA shown in step 5 can act as a template for T7 RNA polymerase-directed transcription (step 6). This amplified RNA product now serves as detection reporter molecules for target sequence and/or serves as additional target molecules to continue the self-cycling reactions (steps 7 through 12).

The most successful reactions, yielding a ca. 10⁶ fold target amplification, functions with three enzymes and two oligonucleotide primers containing the T7 RNA polymerase binding sequence (PBS). The necessary enzymes are AMV reverse transcriptase, T7 RNA polymerase and E. coli RNase H. Addition of E. coli RNase H in the reaction supplements the RNase activity present in AMV reverse transcriptase and appears necessary to produce high levels of amplification. Selection of optimal oligonucleotide primers centers on the areas of the length of the targeted sequence, inclusion of the polymerase binding sequence on one or both primers and efficiency of the polymerase binding sequence-containing primers as a transcription promoter. All three affect the level of amplification. Inclusion of two oligonucleotide primers, each containing a PBS, resulted in more amplification than did inclusion of a single PBS-containing primer and a non-PBS-containing primer.

With reference to the appended drawing:
(1) mRNA target molecule is annealed with a target specific synthetic oligodeoxyribonucloetide incorporating a T7 RNA polymerase promoter binding sequence,
(2) This primer is extended by the DNA polymerase activity of AMV reverse transcriptase to synthesize the first cDNA strand,
(3) The RNase H activity of AMV reverse transcriptase end E. coli RNase H degrade the RNA of the RNA/DNA hybrid duplex, making the DNA available as a template for second-strand cDNA synthesis,
(4) Self-generated oligoribonucloetides resulting from RNase H digestion or a synthetic oligodeoxyribonucleotide preferably incorporating a T7 RNA polymerase promoter binding sequence (not shown in the drawing) prime synthesis of second-strands cDNA. AMV reverse transcriptase then extends the primer to form double-stranded DNA which incorporates an operative T7 RNA polymerase promoter binding sequence,
(5) T7 RNA polymerase binds to the double-stranded promoter binding sequence and transcribes copies of RNA complementary to target nucleic acid,
(6) A second oligodeoxyribonucleotide primer with a PBS anneals to the RNA transcript,
(7) AMV reverse transcriptase catalyses synthesis of a CDNA strand,
(8) RNase H degrades the RNA of the RNA/DNA hybrid duplex and makes the DNA available as a template for second-strand synthesis,
(9) An oligodeoxyribonucleotide primer hybridizes to the second-strand cDNA, and AMV reverse transcriptase synthesizes the DNA. Transcription occurs and cycling continues.

### 4. Examples

### 1. Amplification of a first HIV-1 env region

A region of HIV-1 RNA was amplified in an isothermal enzymatic reaction which generated 10⁶-fold more copies of this region at the end of the reaction.

### a. Amplification reaction

Starting nucleic acid material was cesium-pelleted RNA which had been extracted from HIV-1-infected CEM cells (human lymphoblastoid cell line; Folks et al., Proc. Natl. Sci. USA 82, 4531 (1985)) by the guanidinium isothicyanate-cesium chloride gradient procedure described by Maniatas et at., Supra. (HIV-1-specific sequences were estimated to be 1-10% of the total nucleic acids present.)

One-tenth attomole of target nucleic acid was placed in a reaction mixture (total volume 100 µl) containing (final concentrations):
40 mM Tris.HC1, pH 8.1
12 mM MgC1₂
25 mM NaC1
2 mM Spermidine - (HC1)₃
5 mM Dithiothreitol
80 µg/ml BSA
1 mM each dATPm dCTP, dGTP, dTTP
1 mM each ATP, CTP, GTP, UTP
0.25 µg each priming oligonucleotide (88-211 and 88-347, see infra.)

The reaction components were combined in a 1,5 ml eppendorf tube and then vortexed briefly and lightly. The target nucleic acid was denatured by heating the tube to 65 C for one minute in a water bath. After cooling at 37 C for one minute, the following enzymes were added:
25 units AMV Reverse Transcriptase (15-25 units/µl)
100 units T7 RNA polymerase (100 units/µl)
4 units E. coli RNase H (2 units/µl)

The reaction proceeded for three hours at 37 C with no further manipulation.

### b. Detection of amplification products

After about one hour, the products were detected using a ³²P-labeled oligonucleotide complementary to a 30 basepair region within the amplified fragment (88-298). An aliquot of the reaction, representing 1/40 of the total volume, was denatured in 95.0 µ1 of 7.4% formaldehyde and 10x SSC (Maniatis et al., Supra) at 55 C in a water bath for 20 minutes. Immediately, the aliquot was ice-chilled and then loaded onto a nitrocellulose membrane through a slot-blot apparatus. nucleic acids were immobilized to the nitrocellulose by UV irradiation (254m).

After fixing, the filters were prehybridized at 55 C for 15 minutes in 50-100 µ1 buffer/cm² filter containing 0.5% BSA, 0.5% polyvinylpyrrolidone, 5x SSPE (20x=3.6 M NaCl, 200 mM NaH₂PO₄, 20 mM EDTA, pH 7-8) and 1% SDS. Hybridization occured at 55 C for 2 hours in the same buffer containing 2-5 x 10⁶ cpm/ml of the phosphorylated oligonucleotide probe. The probe was added to the prehybridization buffer. The filters were washed three times at room temperature for three minutes each using 1 ml buffer/cm² filter, 1x SSPE, 1% SDS, then for one minute in the same buffer at 55 C.

The filters were autoradiographed at - 70 C with one intensifying screen.

Levels of amplification were estimated by comparing the intensity of signal produced by the amplified product to the signal produced by known amounts of HIV-1 RNA or pARV7A/2 (Luciw et al. Nature 312, 760 (1984)), a plasmid containing a cDNA copy of the HIV-1 genome inserted into the EcoRI site of pUC19.

In the example described above, the level of amplification was 1 x 10⁶. Northern blot and Southern blot analysis of the product using detection probes 88-297 and 88-298 shows that it was a mixture of DNA and RNA, with RNA as the predominant species. The product was of discrete size (∼210 bp) within a narrow range (20-40 bp).

### 2. Amplification of a Second HIV-1 env Region

Amplification of a second HIV-1 env region was accomplished following the procedures described in Example 1, except priming oligonucleotides 87-284 and 88-347 (infra) were used for amplification, and detection oligonucleotides 86-272 and 86-273 (infra) were used for detection. An amplification of 10³-fold was achieved.

### 3. Amplification of HIV-1 sor region

Amplification of the HIV-1 sor region was accomplished following the procedure described in Example 1, except priming oligonucleotides 88-77 and 87-292 (infra) were used for amplification, and detection oligonucleotides 86-31 and 86-32 (infra) were used for detection. An amplification of 10³-fold was achieved.

### 4. Amplification of the first HIV-1 env region from clinical samples of blood from AIDS patients

RNAs from three HIV-1-infected clinical samples were amplified. RNAs were extracted by the organic extraction protocol (infra).

Amplification was conducted as in Example 1 using priming oligonucleotides 88-211 and 88-347 (infra) and detection oligonucleotides 88-297 and 88-298 (infra).

Two of the samples showed positive results. The total amplification for the reactions in these experiments was 10⁵-fold. Because the signal detected after amplification is directly proportional to the amount of starting material present at the beginning of the reaction (see Example 5), it is possible that the third clinical sample was not identified as being HIV-1-infected because it contained too little starting target HIV-1 sequence.

### 5. Amplification of the first HIV-1 env region in non-infected CEM cells mixed with variable amounts of HIV-1-infected CEM cells

Amplification was conducted as in Example 4 using priming oligonucleotides 88-211 and 88-347, (infra) and detection oligonucleotides 88-297 and 88-298 (infra).

Target amplification using total nucleic acids extracted (see Example 7) from 10³ to 1 HIV-1-infected CEM cells in a population of 10⁶ uninfected CEM cells showed a signal proportional to the number of infected cells present in the sample. The negative control, 10⁶ uninfected CEM cells, showed little background. This background signal was significantly less then the signal obtained from the 10 infected CEM cell sample.

### 6. Reagents and oligonucleotides

- a. Reagents:: Nucleotide triphosphates are from Sigma, AMV Reverse Transcriptase is from Life Sciences, T7 RNA polymerase is from Stratagene, E. coli Ribonuclease H and BSA are from Bethesda Research Labs
- b. Oligos:: Oligonucleotides were synthesized by phosphoramidite chemistry using an Applied Biosystems 380A, then purified by HPLC.
The oligonucleotides used as primers and probes are specific for HIV-1 and correspond to the sequences reported by Ratner et al., Nature 313, 277 (1985), for the env and sor regions.
- 88-211:: (priming oligonucleotide; nt 6450-6479; env) 5'AATTTAATACGACTCACTATA**GGGA**TCTATTGTGCCCCGGCT GTTTTGCGATTCTA-3'
- 88-297:: (detection oligonucleotide; nt 6560-6531; env) 5'-TGGCCTAATTCCATGTGTACATTGTACTGT-3'
- 88-298:: (detection oligonucleotide; nt 6531-6560; env) 5'-ACAGTACAATGTACACATGGAATTAGGCCA-3'
- 88-347:: (priming oligonucleotide; nt 6661-6632; env) 5'AATTTAATACGACTCACTATA**GGGA**TGTACTATTATGGTTTT AGCATTGTCTGTGA-3'
- 88-77:: (NT 5018-4988; sor) priming 5'-AATTTAATACGACTCACTATA**GGGA**CACCTAGGGCTAACTAT GTGTCCTAATAAGG-3'
- 87-292:: (nt 4766-4796; sor) priming 5'-TAATACGACTCACTATA**GGGA**AAGAATAAGTTC AGAAGTACACATCCCACT-3'
- 86-31:: (nt 4901-4932; sor detection 5'-GCACACAAGTAGACCCTGAACTAGCAGACCA-3'
- 86-32:: (nt 4932-4901; sor) detection 5'-TGGTCTGCTAGTTCAGGGTCTACTTGTGTGC-3'
- 87-284:: (nt 6551-6579; env) priming 5'-TAATACGACTCACTATA**GGGA**AATTAGGCCAGT AGTATCAACTCAACT-3'
- 86-272:: (nt 6591-6620; env) detection 5'-TCTAATTACTACCTCTTCTTCTGCTAGACT-3'
- 86-273:: (nt 6620-6591; env) detection 5'-AGTCTAGCAGAAGAAGAGGTAGTAATTAGA-3'

Each priming oligonucleotide contains a sequence at the 5' end which is the T7 RNA polymerase binding sequence (underlined) and the preferred transcriptional initiation site (bold). The remainder of the sequence is complementary to the target sequence.

Relative to the HIV-1 target sequence, oligonucleotides 88-211, 88-298, 87-292, 86-31, 87-284, and 86-273 are complementary to the negative strand, and oligonucleotides 88-347, 88-297, 88-77, 86-32, and 86-272 are complementary to the positive strand.

### 7. Organic Extraction of RNAs

Protocol for nucleic acid preparation on infected cell samples:
Pellet cells: 5k rpm for 10' from 1 ml Tris-buffered saline. Draw off and discard supernatant, leaving 50 µl with pellet.
Resuspend pellet in 600 µl Lysis Buffer.
Vortex vigorously and incubate at 50 C for 45', vortexing for 10-15s every 10'.
Add 600 µl phenol:chloroform:isoamyl alcohol (50:48:2). Shake and vortex to emulsify mixture. Centrifuge at 14K rpm for 2' to separate phases.
Draw off 575 µl from aqueous (top) phase. Add 600 µl phenol:chloroform:isoamyl alcohol (50:48:2). Shake and vortex to emulsify mixture. Centrifuge at 14K rpm for 2' to separate phases.
Draw off 525 µl from aqueous phase. Add 600 µl chloroform:isoamyl alcohol (24:1). Shake and vortex to emulsify mixture. Centrifuge at 14K rpm for 2' to separates.
Draw off 400 µl from aqueous phase. (Do not transfer any cell debris which may be at the interface.)
Add 1/10 volume (40 µl) 8 M LiCl. At this step, samples may be split for processing.
Add 3 volumes ice cold 100% ethanol to samples which have been split. Add 2.5 volumes ice cold 100% ethanol to samples which have not been split. Mix well. Precipitate at -20 C overnight or in a dry ice/ethanol bath for 15'.

### Reagents

### Lysis Buffer

20 mM Tris pH 7.5
150 mM NaCl
10 mM EDTA
0.2% SDS
200 µg/ml proteinase K

### Tris-buffered Saline

137 mM NaCl
5.1 mM KCl
24.8 mM Tris base
   Adjust pH to 7.4 with l N HCl
   Sterilize by autoclaving.

The foregoing description details specific methods that can be employed to practice the present invention. Having detailed specific methods initially used to describe the isothermal amplification system hereof, the art skilled will well enough know how to devise alternative equivalent methods resulting in similar amplification in an isothermal single-pot setting and for extending this information to target nucleic acids other than those specifically disclosed. Thus, however detailed the foregoing may appear in text, it should not be construed as limiting the overall scope hereof; rather, the ambit of the present invention is to be governed only by the lawful construction of the appended claims.

## Claims

1. A method of preparing a double-stranded DNA encoding a sequence corresponding to a target nucleic acid sequence and having an operative polymerase promoter, comprising:
a) providing a first DNA primer containing a RNA polymerase promoter sequence operatively associated with a sequence that is a complement of a segment of a target nucleic acid sequence,
b) contacting under suitable hybridizing conditions said first DNA primer with a nucleic acid sample that may contain said target nucleic acid sequence,
c) permitting primer extension of any hybridization product of said first DNA primer with said target nucleic acid sequence in a DNA polymerase extension reaction to form a corresponding RNA/DNA duplex nucleic acid,
d) selectively digesting enzymatically the RNA strand of said RNA/DNA duplex nucleic acid,
e) permitting hybridization to the freed promoter containing cDNA strand under suitable hybridization conditions of a second nucleic acid primer, said second nucleic acid primer being a product of said selective digestion or being an extrinsically derived oligonucleotide primer optionally bearing operatively a promoter sequence, and
f) permitting primer extension of hybridization product of primer with said DNA strand in a DNA polymerase extension reaction.

2. A method useful for the detection of at least one specific target nucleic acid sequence in a nucleic acid sample that may contain said nucleic acid target sequence, comprising:
g) employing the prepared double-stranded DNA of Claim 1 as a double-stranded DNA template for the preparation of a plurality of RNA transcripts therefrom, each bearing a RNA sequence corresponding to said target nucleic acid sequence.

3. The method according to Claim 2 comprising the additional step of detecting and optionally measuring the presence of said RNA sequence.

4. The method according to any one of Claims 1, 2, or 3 wherein said target nucleic acid sequence in said nucleic acid sample is present intrinsically as such or is a corresponding DNA target sequence extrapolation product prepared by transcription from a double-stranded DNA template prepared by polymerase based primer extension of a primer hybridized to a separated DNA strand operatively bearing a promoter sequence prepared from a hybridization/primer extension product of said DNA target sequence with a primer operatively bearing a promoter sequence.

5. The method according to Claim 2 comprising the additional steps of
h) permitting hybridization of said RNA transcripts under suitable hybridization conditions with a DNA primer containing a promoter sequence operatively associated with a sequence that is a complement of a segment of said RNA transcript sequence,
i) permitting primer extension of hybridized product of step h) in a DNA polymerase extension reaction to form a corresponding RNA/DNA duplex nucleic acid,
j) selectively digesting enzymatically the RNA strand of said RNA/DNA duplex nucleic acid of step i),
k) permitting hybridization of the freed promoter containing DNA strand product of step j) under suitable hybridization conditions with a nucleic acid primer, said nucleic acid primer being a product of said selective digestion of step j) or being an extrinsically derived oligonucleotide primer optionally bearing operatively a promoter sequence,
l) permitting primer extension of hybridization product of step k) in a DNA polymerase extension reaction, and
m) employing the double-stranded DNA product of step l) as a double-stranded DNA template for the preparation off a plurality of RNA transcripts therefrom.

6. The method according to Claim 5 wherein the RNA transcript products have a sense opposite those RNA transcript products of Claim 2.

7. The method according to Claim 5 permitted to proceed continuously and spontaneously by presence in the reaction milieu of enzyme activities provided by:
1) a reverse transcriptase, 2) an enzyme having RNase H activity, 3) a DNA-dependent RNA polymerase and 4) at least one oligonucleotide primer sequence operatively bearing a promoter sequence.

8. The method according to Claim 7 conducted substantially isothermally.

9. A method comprising employing the double-stranded nucleic acid according to Claim 1 or step l) of Claim 5 as a template for the preparation of a plurality of RNA transcripts therefrom in a reaction catalyzed by a polymerase that recognizes the promoter thereof, each bearing a RNA sequence corresponding to said target nucleic acid sequence, and detecting and optionally measuring the presence of said RNA transcripts.

10. The method according to Claim 2 or 9 wherein said transcripts contain RNA replicase recognition site for replication off said transcripts by RNA replicase induction.

11. The method according to Claim 2, 9, or 10 wherein the detected RNA sequence of said RNA transcripts is measured in a standardized manner so as to measure the amount of target nucleic acid sequence contained in a sample of nucleic acid used in preparing the double-stranded nucleic acid template.

12. The method according to Claim 11 wherein the detected RNA sequence of said RNA transcripts is measured in a manner internally standardized with the presence of a known copy number of nucleic acid also contained in said sample.

13. The method according to Claim 1 wherein said target nucleic acid sequence is associated with the characteristics of a genetic or pathogenic disease or condition.

14. The method according to Claim 1 wherein said target nucleic acid sequence is associated with a human immunodeficiency virus.

15. The method according to Claim 1 wherein said target nucleic acid sequence is associated with a defective gene.

16. The method according to Claim 1 or 5 wherein the promoter is a bacteriophage T7 promoter and the RNA transcripts are produced using T7 RNA polymerase.

17. The method according to Claim 1 or 5 wherein the selective digestion is conducted with RNase H enzyme.

18. The method according to Claim 1 or 5 wherein the extension reaction is catalyzed by E. coli DNA polymerase I.

19. The method according to Claim 1 or 5 wherein the extension reaction is catalyzed by Klenow fragment of E. coli DNA polymerase I.

20. The method according to Claim 1 or 5 wherein the extension reaction is catalyzed by T7 DNA polymerase.

21. The method according to Claim 1 or 5 wherein the extension reaction is catalyzed by reverse transcriptase.

22. The method according to Claim 2 or 5 wherein said RNA transcripts are labelled prior to detection.

23. The method according to Claim 22 wherein said RNA transcripts are radio-labelled.

24. The method according to Claim 22 wherein said RNA transcripts are chromophore labelled.

25. The product of the process which comprises treating a RNA/DNA duplex RNA with an enzyme having RNase H activity, wherein the DNA strand has at its 5'-end an operative promoter sequence.

26. The product according to Claim 25 wherein said enzyme is RNase H.

27. In a method of amplifying a target nucleic acid sequence, generated as such or as an extrapolation product from a corresponding target DNA sequence, which method comprises the steps of hybridization with a DNA primer having a promoter sequence operatively associated therewith followed by polymerase primer extension to give a corresponding RNA/DNA duplex and hybridization of the said DNA extension product strand bearing the promoter sequence of said duplex with a second primer followed by polymerase primer extension to form a double-stranded DNA template useful for preparation of optionally replicatable RNA transcripts therefrom for detection as such or for further use in reactions as defined above, the improvement wherein said DNA extension product strand bearing the promoter sequence of the said RNA/DNA duplex is made available for the following hybridization thereof with said second primer by the selective digestion enzymatically of the RNA strand of said RNA/DNA duplex with an enzyme having the activity of RNase H enzyme.

28. The improvement according to Claim 27 wherein said enzyme is RNase H.

29. A kit useful for the detection of at least one specific nucleic acid target sequence in a RNA sample that may contain said nucleic acid target sequence, comprising a reaction mixture comprising a reverse transcriptase, an enzyme having RNase H activity, a DNA-dependent RNA polymerase and at least one DNA primer containing a promoter sequence operatively associated with a sequence complementary to a segment of a target nucleic acid sequence or its complement and means whereby upon contact of said mixture with sample for detection of target nucleic acid sequence, target nucleic acid sequence is detected after amplification.

30. The kit according to Claim 29 having means to conduct reactions involving its components at isothermal conditions.

31. The kit according to Claim 30 wherein said enzyme having RNase H activity is RNase H.
